**Europäisches Patentamt**

**European Patent Office**

**Office européen des brevets**

(19)

(11) **EP 1 452 862 A1**

(12) **EUROPÄISCHE PATENTANMELDUNG**

(43) Veröffentlichungstag:
    **01.09.2004 Patentblatt 2004/36**

(51) Int Cl.⁷: **G01N 27/414**

(21) Anmeldenummer: **04100809.5**

(22) Anmeldetag: **01.03.2004**

(84) Benannte Vertragsstaaten:
    **AT BE BG CH CY CZ DE DK EE ES FI FR GB GR
    HU IE IT LI LU MC NL PL PT RO SE SI SK TR**
    Benannte Erstreckungsstaaten:
    **AL LT LV MK**

(30) Priorität: **28.02.2003 DE 10308882**

(71) Anmelder: **SIEMENS AKTIENGESELLSCHAFT**
    **80333 München (DE)**

(72) Erfinder:
    • **Dellemann, Jean-Philippe**
      **81825, München (DE)**

• **Fleischer, Maximilian**
  **85635, Höhenkirchen (DE)**
• **Lampe, Uwe**
  **21614, Buxtehude (DE)**
• **Meixner, Hans**
  **85540, Haar (DE)**
• **Pohle, Roland**
  **85570, Herdweg (DE)**
• **Simon, Elfriede**
  **80639, München (DE)**

(54) **Gassensitiver Feldeffekttransistor mit einer gassensitiven Schicht und Verfahren zu deren Herstellung**

(57) Gassensitiver Feldeffekttransistor (FET) mit einer gassensitiven Schicht, mit der Sensorsignale durch Messung der Änderung der Austrittsarbeit generierbar sind, wobei die gassensitive Schicht aus Bariumtitanat ($BaTiO_3$) mit einem Überschuss an Bariumoxid (BaO) besteht und ein Teil der Schicht oberflächlich als Bariumcarbonat ($BaCO_3$) ausgebildet ist.

FIG 1

$BaTiO_3/CuO//Raumtemperatur$

1) —— relative Feuchte in % 100    2) —— CO2 Fluß in ml/1000    3) —— Sensorsignal in Volt

EP 1 452 862 A1

**Beschreibung**

Die Erfindung betrifft einen gassensitiven Feldeffekttransistor, der mit einer gassensitiven Schicht ausgestattet ist, um durch die Auswertung der Änderung der Austrittsarbeit an der gassensitiven Schicht bestimmte Zielgase zu detektieren.

Als Beispiel für ein Zielgas wird beispielsweise Kohlendioxid ($CO_2$) detektiert. Die Messung von Kohlendioxid, entweder als Schwellwert oder als Konzentration, wird in vielen Bereichen des täglichen Lebens benötigt. Wegen seiner Eigenschaft als chemisch relativ inaktives Gas gibt es zur Zeit nur aufwändige und kostspielige Messsysteme wie elektrochemische Zellen oder dispersive oder nichtdispersive Infrarotspektrometer. Eine kostengünstige Messung von Kohlendioxid mit einfachen Gassensoren ist zur Zeit noch nicht möglich. Gasempfindliche Feldeffekttransistoren mit kohlendioxid-sensitiven Materialien können ein Weg sein, dies zu realisieren.

Gassensitive Feldeffekttransistoren können beispielsweise folgende Vorteile aufweisen:

- Der Betrieb bei Raumtemperatur und geringfügig darüber erlaubt den Einsatz in Bereichen, in denen nur ein geringer Energieverbrauch durch Sensoren zulässig ist, wie beispielsweise beim Batteriebetrieb, in explosionsgeschützten Bereichen, bei direktem Anschluss an Datenbusse ohne zusätzliche Hilfsenergie für sogenannte Niedrigenergie-Sensoren (Low-Power-Sensors).
- Eine vollständige Kompatibilität der Herstellung des Bauelementes mit der eingeführten Siliziumtechnologie.
- Eine einfache Realisierung von Sensor-Arrays mit unterschiedlich gasempfindlichen Sensoren zum Ausschluss von Querempfindlichkeiten oder zum Aufbau von elektronischen Nasen.
- Vollständige bzw. teilweise Integration des Sensors in seine Auswerte-Elektronik durch monolithischen Aufbau.

Low-Power-Kohlendioxidsensoren können beispielsweise Anwendung finden als Schwellwertsensoren zur Raumluftüberwachung, beispielsweise in Räumen mit Klimaanlagen, die mit Kohlendioxid als Kältemittel betrieben werden, in Gärkellern, in Lagerräumen für landwirtschaftliche Produkte oder im Bergbau. Als Einzelelemente für Sensor-Arrays kommt beispielsweise der Bereich der Brandüberwachung, der Atemluftüberwachung oder der Raumluftqualität in Frage.

Die Messung von Kohlendioxid wurde bislang lediglich mit äußerst aufwändigen Messsystemen ermöglicht. Sehr häufig ist der Einsatz von elektrochemischen Zellen, die im Vergleich zu anderen Messsystemen noch am günstigsten sind, jedoch eine begrenzte Lebensdauer haben. Für Messungen unter erhöhten Anforderungen wie beispielsweise Genauigkeit und Messbereich, werden zumeist Infrarotspektrometer benutzt. Ein weiteres optisches Verfahren ist der Einsatz von Infrarot-Laserdioden, was ebenfalls keine kostengünstige Variante der Kohlendioxid-Messung ist.

Beim Sensormaterial wird im Stand der Technik von einer Mischung aus Bariumtitanat mit Kupferoxid im Molverhältnis von 1:1 ausgegangen. Das Material wird beispielsweise zu Presslingen verarbeitet, [1]. Die Gassensitivität wird anschließend bei höheren Temperaturen bei ca. 450°C über die Änderung der elektrischen Kapazität des Presslings in Abhängigkeit von der Kohlendioxidkonzentration in der Gasphase gemessen.

In [2] wird ähnlich wie in [1] über eine Verbesserung der Sensitivität durch Hinzufügen von Additiven wie $ZnO_2$, $SrO_2$, $La_2O_3$ oder Ag im Bereich von etwa 1-2 Gewichtsprozent berichtet. In einer weiteren Veröffentlichung [3] wird ein Mechanismus für die Gassensitivität vorgeschlagen. Danach wird Folgendes berichtet: Kupferoxid ist ein p-Halbleiter, Bariumtitanat ist ein n-Halbleiter. An den Übergängen zwischen beiden Halbleitermaterialien sollen Raumladungszonen vorhanden sein, wobei sich durch Rekombination von Löchern des Kupferoxids mit den Elektronen des Bariumtitanats zu beiden Seiten des Materialkontaktes Verarmungszonen ausbilden. Diese haben wegen der geringen Ladungsdichte im Bariumtitanat eine größere Ausdehnung im Kupferoxid. Unter Luft soll Sauerstoff auf beiden Metalloxiden adsorbiert werden, wobei dieser Elektronen bei einer Adsorption auf Bariumtitanat bzw. Löcher bei einer Adsorption auf Kupferoxid bindet. Durch die Adsorption von Kohlendioxid soll adsorbierter Sauerstoff verdrängt werden, wodurch sich die Elektronendichte im Bariumtitanat erhöht. Dies soll unmittelbaren Einfluss auf die Ausdehnung der Raumladungszone haben und könnte als Änderung der Kapazität des Materialgemisches gemessen werden.

In [4] wird die Herstellung einer gassensitiven Schicht als siebgedruckte Dickschicht beschrieben. Die Auslesung der Gassensitivität erfolgt über die Messung des Sensorwiderstands im Temperaturbereich zwischen 450 -600°C mit einem Maximum der Sensitivität bei 550°C. Durch Additive kann sich angeblich die Empfindlichkeit des $CO_2$ erhöhen.

Die in sämtlichen Literaturstellen beschriebenen Sensoren müssen alle bei höheren Temperaturen von mindestens 450°C betrieben werden und sind sowohl wegen ihres hohen Energiebedarfs als Niedrigenergie-Sensoren (Low-Power-Sensor) als auch wegen ihrer hohen Betriebstemperatur für eine monolithische Integration in die Elektronik ungeeignet. Eine Auslesung derartiger Materialsysteme über eine Messung der Änderung der Austrittsarbeit wurde bisher noch nicht untersucht.

Für einen Low-Power-Sensor wurde Bariumcarbonat in der deutschen Patentanmeldung DE 198 148 56.9 als Dickschicht untersucht. Dabei wird die Änderung des Kontaktpotenzials (Austrittsarbeit) mit Hilfe von Kelvin-Sonden in Abhängigkeit von der Kohlendioxid-Konzentration des Gases als Messsignal benutzt. Das Material zeigt bereits bei Raumtemperatur eine deutliche Gassensitivität. Als gassensitive Grundreaktion wird ein Gleichgewicht zwischen Carbonat und Hydrogencarbonat angesetzt:

$$BaCO_3 + CO_2 + H_2O \Leftrightarrow Ba\,(HCO_3)_2$$

Entsprechend dieses Gleichgewichts kann die Reaktion nur bei gleichzeitiger Anwesenheit von Luftfeuchtigkeit und Kohlendioxid ablaufen. Durch Fehlen einer dieser Komponenten zersetzt sich das nur in geringen Mengen vorhandene Bariumhydrogencarbonat $Ba(HCO_3)_2$ sehr schnell vollständig.

Messungen mit Bariumkarbonat-Dickschichten bestätigen, dass eine sensitive Reaktion auf Kohlendioxid nur in Anwesenheit von Feuchtigkeit möglich ist. Ein längeres Verweilen einer sensitiven Schicht in trockener, kohlendioxidfreier Atmosphäre führt sehr schnell zu einer Deaktivierung des Sensormaterials. Bei der Anwendung von Bariumkarbonat als kohlendioxidsensitive Schicht im gassensitiven Feldeffekttransistor treten bisher folgende Probleme auf:

- die Haftung der Bariumkarbonat-Dickschichten ist sehr schlecht und erlaubt kaum einen sicheren Einbau in einem gassensitiven Feldeffekttransistor.
- Die Ansprechzeit ist zu hoch und liegt weit über einer Minute bei Raumtemperatur.
- Die Schicht deaktiviert nach mehreren Wochen sehr stark, kann jedoch durch Erwärmung auf über 100°C unter feuchter, kohlendioxidhaltiger Luft reaktiviert werden.

Der Erfindung liegt die Aufgabe zugrunde, eine dauerhafte und auf ein Zielgas, insbesondere Kohlendioxid, hoch sensitive, gassensitive Schicht bereitzustellen, die in einem gassensitiven Feldeffekttransistor als gassensitive Schicht einsetzbar ist. Weiterhin soll die Aufgabe die reproduzierbare Herstellung der gassensitiven Schicht umfassen. Die Lösung dieser Aufgabe geschieht jeweils durch den Gegenstand der Patentansprüche 1 bzw. 5. Vorteilhafte Ausgestaltungen können den Unteransprüchen entnommen werden.

Der Erfindung liegt die Erkenntnis zugrunde, dass die Adsorption eines Zielgases an der gassensitiven Schicht durch Bariumtitanat gewährleistet ist, durch welches gleichzeitig eine ausreichende Stabilität der gassensitiven Schicht erzielbar ist. Eine ein Messsignal erzeugende Gleichgewichtsreaktion ist am Beispiel des Zielgases Kohlendioxid die Reaktion zwischen Bariumkarbonat und Bariumhydrogencarbonat in Anwesenheit von Feuchtigkeit, insbesondere Luftfeuchtigkeit. Somit sind sowohl die Adsorption als auch die Detektion bei ausreichender Sensitivität an einer gassensitiven Schicht gewährleistet. Die gassensitive Schicht ist nun in einem Feldeffekttransistor integriert und es werden Sensorsignale generiert, die auf dem Prinzip der Messung der Änderung der Austrittsarbeit basieren. Die Ansprechzeit des Sensors ist ausreichend kurz und liegt ca. bei 1 Minute. Der Sensor kann bei Raumtemperatur betrieben werden.

Die Präparation der gassensitiven Schicht erfolgt auf der Grundlage von Bariumtitanat mit einem Überschuss an Bariumoxid. Eine daraus hergestellte Schicht wird in kohlendioxidhaltiger Atmosphäre getempert. Durch die Reaktion des Kohlendioxids bei hohen Temperaturen von mindestens 500°C wird ein Teil des überschüssigen Bariumoxids zu Bariumkarbonat umgesetzt. Somit liegt eine Schicht aus im Wesentlichen Bariumtitanat vor, die partiell mit Bariumkarbonat durchsetzt oder behaftet ist. Bei einer späteren Messung kann in Anwesenheit von Feuchtigkeit der Anteil von Kohlendioxid in der umgebenden Atmosphäre gemessen werden.

Alternativ kann die gassensitive Schicht als Dünnschicht ausgebildet werden. Ein hierzu verwendetes Abscheideverfahren ist beispielsweise das PVD-Verfahren (Physical Vapour Deposition) oder die Kathodenzerstäubung (Sputtern).

Eine vorteilhafte Ausgestaltung der Erfindung sieht vor, dass zur Verbesserung der Gassensitivität dem Schichtmaterial bestehend aus Bariumtitanat mit einem Überschuss an Bariumoxid Kupferoxid in einem Molverhältnis von 1:1 beigemischt ist. Das Tempern der Schicht zur Aktivierung der Gassensitivität in einer Kohlendioxidatmosphäre wird auch hier vorgenommen. Bei der Aktivierung wird überschüssiges Bariumoxid bzw. ein Teil des Kupferoxids zu Bariumcarbonat umgesetzt. Ausgewiesen wird die gassensitive Reaktion der $BaTiO_3$- bzw. $BaTiO_3/CuO$-Dickschicht mittels der Änderung der Austrittsarbeit.

Im Folgenden werden in Verbindung mit den Figuren Ausführungsbeispiele beschrieben, die spezielle Ausgestaltungen der Erfindung wiedergeben.

Fig. 1    zeigt ein Sensorsignal, das der Austrittsarbeitsdifferenz beim Wechsel einer Kohlendioxidkonzentration bei Raumtemperatur entspricht,

Fig. 2    zeigt ein Sensorsignal, das der Austrittsarbeitsdifferenz beim Wechsel einer Kohlendioxidkonzentration bei 150°C Betriebstemperatur entspricht.

Die Grundlage für das gassensitive Material ist ein Bariumtitanat mit einem Überschuss an Bariumoxid. Aus dem Pulver wird durch Zufügen von einem Binder eine druckfähige Paste hergestellt, die durch Siebdruck zu einer Dickschicht verarbeitet wird, die mehrere Stunden unter Luft zum Ausbrennen des Binders und anschließend unter Kohlendioxidatmosphäre getempert wird. Durch die Reaktion unter Kohlendioxid bei hohen Temperaturen von mehr als 500°C wird ein Teil des überschüssigen Bariumoxids zu Carbonat bzw. Bariumcarbonat umgesetzt. In Anwesenheit von Feuchtigkeit können sich Hydrogencarbonate bilden, so dass der Anteil des Kohlendioxids in der umgebenden Atmosphäre sich vergrößert. Alternativ kann die gassensitive Schicht als Dünnschicht präpariert werden, beispielsweise mit einem Verfahren wie der Kathodenzerstäubung.

Eine Weiterbildung der Erfindung sieht zur Verbesserung der Gassensitivität vor, dass dem Grundmaterial der gassensitiven Schicht Bariumtitanat mit Bariumoxid im Überschuss Kupferoxid im Molverhältnis 1:1 beigemischt wird. Die Pulvermischung wird mit Ethylcellulose zu einer druckfähigen Paste verarbeitet. Das Einbrennen der Dickschicht erfolgt unter Luft, woraufhin eine Aktivierung unter Kohlendioxidatmosphäre erfolgt. Bei dieser Aktivierung wird das überschüssige Bariumoxid bzw. ein Teil des Kupferoxids zu Carbonat bzw. Bariumcarbonat umgesetzt. An diesem Material können daher beide oben bereits erwähnten Reaktionen mit Kohlendioxid ablaufen:

- die Adsorption des Zielgases am Bariumtitanat,
- die Gleichgewichtsreaktion zum Hydrogencarbonat in Anwesenheit von Luftfeuchte, wenn das Zielgas Kohlendioxid ist. Die Sensoreigenschaften können durch Additive im Bereich von 1-3 Gewichtsprozent weiter verbessert werden. Als Additive kommen u. a. $La_2O_3$, SrO, ZnO, $CaCO_3$ in Frage.

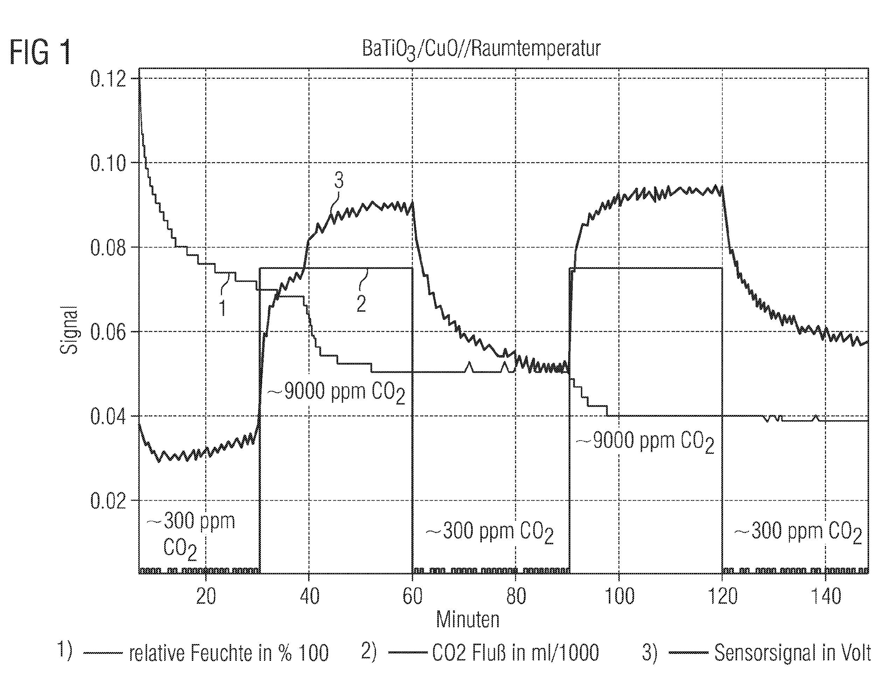

Ausgelesen wird die gassensitive Reaktion der Bariumtitanat- bzw. der Bariumtitanat/Kupferoxid-Dickschicht über die Änderung der Austrittsarbeit. Ein Beispiel für Kohlendioxidmessungen wird in Fig. 1 wiedergegeben. Die Messung wird vorzugsweise mit Hilfe der Kelvinsonde ausgeführt, wobei die gassensitive Schicht auf Raumtemperatur war. Sauerstoffgehalt und Luftfeuchte wurden bei der Messung konstant gehalten. Zu sehen ist eine schnelle Reaktion des Sensormaterials auf die Konzentrationsänderung mit einer kurzen Ansprechzeit von ca. einer Minute.

Die Erfindung erzielt, insbesondere folgende Vorteile:

1. Die Haftung der BaCi03- bzw. BaCiO3/CuO-Dickschicht ist gegenüber der Bariumcarbonat ($BaCO_3$)-Dickschicht deutlich verbessert. Damit können die erforderlichen Schritte der Fertigung des gassensitiven Feldeffekttransistors ohne Ausbeuteverluste durch Ablösen bzw. teilweises Ablösen der Sensorschicht beim Aufbauprozess durchgeführt werden.

2. Bei tiefen Temperaturen (Raumtemperatur) ist die Gassensitivität höher und die Ansprechzeit deutlich niedriger als beim Bariumcarbonat.

3. Gegenüber der kapazitiven bzw. resistiven Auslegung des Gassignals der gassensitiven Schicht ergibt sich die Möglichkeit bei tiefen Temperaturen zu messen. Damit kann ein Low-Power-Sensor realisiert werden.

4. Eine Integration dieser sensitiven Schichten in monolithisch aufgebaute Sensor-Arrays und in die Sensorelektronik erscheint als leicht möglich.

5. Eine Herstellung der gassensitiven Schicht mit Hilfe der Dünnschicht-Technologie ist möglich. Damit können Schichten mit geringeren Schichtdicken und Korngrößenverteilungen hergestellt werden.

6. Die Herstellung des Bariumtitanat-Pulvers mit Bariumoxid-Überschuss:
Es werden Titandioxid und Bariumcarbonat-Pulver in einem molaren Verhältnis gemischt, so wie der Bariumoxid-Überschuss eingestellt werden soll. Für ein Bariumtitanat mit 5 Mol % Bariumoxid-Überschuss wird von einer Menge von 1 Mol Titandioxid und 1,05 Mol Bariumcarbonat ausgegangen. Die Pulver werden so gut wie möglich vermischt und anschließend 15 Stunden bei 1300°C gesintert. Die Sintermasse wird gemahlen und anschließend noch einmal 15 Stunden bei 1300°C getempert.

Ein derart hergestelltes Bariumtitanat-Pulver wird mit Kupferoxid (CuO)-Pulver im Molverhältnis 1:1 gut vermischt. Anschließend erfolgt keine Wärmebehandlung. Zunächst wird bei jedem hergestellten Pulver eine Mischung mit einem Binder, in diesem Fall Ethylcellulose/Terpineol-Gemisch, hergestellt. Vorteilhaft hat sich ein Gemisch von 50 Gewichtsprozent Pulver mit 50 Gewichtsprozent Binder herausgestellt. Anschließend wird die Paste im Walzenstuhl oder durch längere Behandlung von mindestens 1 Stunde mit Ultraschall homogenisiert.

Dickschichten werden durch Siebdrucke hergestellt. Als Substrat wird Aluminiumoxid-Keramik mit einem Reinheitsgrad von 99,9% benutzt. Der elektrische Anschluss der Schicht erfolgt über eine Platinelektrode. Die gedruckten Schichten werden zunächst getrocknet (1 Stunde bei 230°C), um das Terpineol auszutreiben und anschließend 4 Stunden lang bei 600°C unter Luft eingebrannt. Dabei verbrennt die Ethylcellulose. Anschließend erfolgt eine Aktivierung der Schicht in Kohlendioxid-Atmosphäre (4 Stunden bei 600°C). Reine Bariumtitanat-Schichten können abweichend auch bei 800 °C eingebrannt und bei 700 °C unter Kohlendioxid-Atmosphäre aktiviert werden.

Zur Charakterisierung der gassensitiven Eigenschaften einer erfindungsgemäßen gassensitiven Schicht können Kontaktpotenzialmessungen bzw. Änderungen in der Austrittsarbeit mit der Kelvinsonde durchgeführt werden. Dabei werden für die charakterisierenden Messungen der Kohlendioxidempfindlichkeit Messungen in synthetischer Luft bei definierter Feuchte durchgeführt. Die Temperatur der gasempfindlichen Schicht konnte über eine auf die Rückseite des Keramiksubstrates gedruckte Platinheizwendel zwischen Raumtemperatur und 280°C eingestellt werden. Die Messergebnisse zeigen die Graphen 1 und 2.

In Fig. 1 sind drei unterschiedliche Signale gegen die Zeit aufgetragen. Die Kurve 1 stellt den Verlauf der relativen Feuchte dar. Kurve 2 gibt den zeitlichen Verlauf der höheren Kohlendioxidkonzentrationen an. Die Kurve 2 gibt das eigentliche Sensorsignal wieder, welches ohne wesentliche Verzögerungen bei einer Kohlendioxidkonzentration von 9000 ppm auf die entsprechende Kohlendioxidkonzentration anspricht. Der Konzentrationshub des Kohlendioxids geht in diesem Fall von 300 ppm auf 9000 ppm.

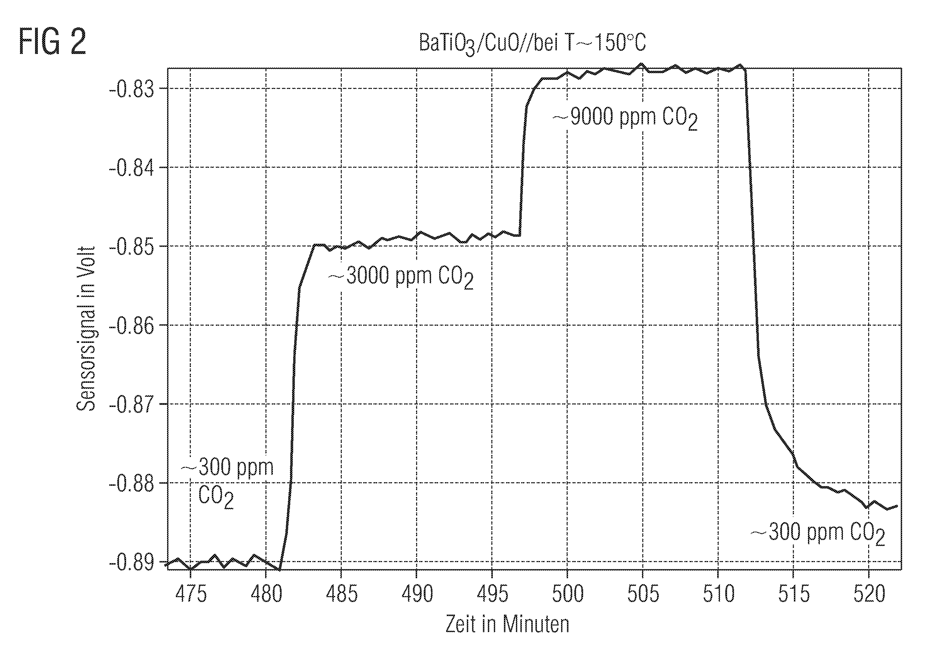

Fig. 2 zeigt ein Sensorsignal, bei dem der Sensor auf 150 °C Betriebstemperatur gehalten wird. Erkennbar ist, dass, ausgehend von einer Konzentration von 300 ppm $CO_2$ nach einem Konzentrationshub auf 3000 ppm $CO_2$ eine eindeutige Signalveränderung erkennbar ist. Ein weiterer Konzentrationshub von 3000 ppm auf 9000 ppm $CO_2$ lässt sich ebenfalls klar darstellen.

Literaturliste

[1] T. Ishihara et al.:"Application of mixed oxide capacitor to the selective carbon dioxide sensor", J. Electrochem.Soc., Vol. 138, 1991, p. 173-176

[2] T. Ishihara et al., "Improved sensitivity of $CuO-BaTiO_3$ capacitive-type $CO_2$ sensor by additives", Sensors and Actuators B28, 1995, 49-54

[3] T. Ishihara et al., "Detection Mechanism of $CuO-BaTiO_3$ capacitive type C02 sensor", Electrochemical Society Proceedings, Vol 96-27, p. 123-130

[4] A. Haeusler, J.U. Meyer, "A novel thick film conductive type $CO_2$ sensor", Sensors and Actuators B34, 1996, p. 388-395

**Patentansprüche**

1. Gassensitiver Feldeffekttransistor (FET) mit einer gassensitiven Schicht, mit der Sensorsignale durch Messung der Änderung der Austrittsarbeit generierbar sind, wobei die gassensitive Schicht aus Bariumtitanat ($BaTiO_3$) mit einem Überschuss an Bariumoxid (BaO) besteht und ein Teil der Schicht oberflächlich als Bariumcarbonat ($BaCO_3$) ausgebildet ist.

2. Gassensitiver Feldeffekttransistor (FET) nach Anspruch 1, bei dem die gassensitive Schicht aus Bariumtitanat (Ba-$TiO_3$) mit Bariumoxid (BaO)-Überschuss besteht, im MolVerhältnis 1:1 Kupferoxid (CuO) enthält und ein Teil der Schicht oberflächlich als Bariumcarbonat ($BaCO_3$) ausgebildet ist.

3. Gassensitiver Feldeffekttransistor (FET) nach Anspruch 1, bei dem die gassensitive Schicht aus Bariumtitanat (Ba-$TiO_3$) ohne Bariumoxid (BaO)-Überschuss besteht, im MolVerhältnis 1:1 Kupferoxid (CuO) enthält und ein Teil der Schicht oberflächlich als Bariumcarbonat ($BaCO_3$) ausgebildet ist.

4. Gassensitiver Feldeffekttransistor (FET) nach einem der vorhergehenden Ansprüche, bei dem in der gassensitiven Schicht Additive mit einem Anteil im Bereich von 1-3 Gewichtsprozent in Form von La2O3, SrO, ZnO oder CaCO3 enthalten sind.

5. Gassensitiver Feldeffekttransistor (FET) nach einem der vorhergehenden Ansprüche, bei dem die gassensitive Schicht eine getemperte Dünn- oder Dickschicht mit Anteilen von Bariumcarbonat ($BaCO_3$) ist.

6. Verfahren zur Herstellung einer gassensitiven Schicht in einem gassensitiven Feldeffekttransistor, bei dem

   - Bariumtitanatpulver mit Bariumoxid-Überschuss mit Binder gemischt wird, so dass eine Paste entsteht,
   - durch Siebdruck hergestellte Dickschichten, mehrere Stunden unter Luft und anschließend unter Kohlendioxid-Atmosphäre bei Temperaturen von mehr als 500 °C getempert werden, wobei Bariumoxid zu Bariumcarbonat umgewandelt wird.

7. Verfahren zur Herstellung einer gassensitiven Schicht in einem gassensitiven Feldeffekttransistor, bei dem eine Dünnschicht aus Bariumtitanat mit Bariumoxid-Überschuss in einem physikalischen Abscheide-Verfahren, insbesondere Kathodenzerstäubung, hergestellt wird und diese Dünnschicht mehrere Stunden unter Luft und anschließend unter Kohlendioxid ($CO_2$) bei Temperaturen von mehr als 500 °C getempert werden, wobei Bariumoxid zu Bariumcarbonat umgesetzt wird.

FIG 1

BaTiO$_3$/CuO//Raumtemperatur

~9000 ppm CO$_2$

~9000 ppm CO$_2$

~300 ppm CO$_2$

~300 ppm CO$_2$

~300 ppm CO$_2$

Minuten

1) —— relative Feuchte in % 100     2) —— CO2 Fluß in ml/1000     3) —— Sensorsignal in Volt

EP 1 452 862 A1

FIG 2

BaTiO$_3$/CuO//bei T~150°C

~9000 ppm CO$_2$

~3000 ppm CO$_2$

~300 ppm CO$_2$

~300 ppm CO$_2$

Sensorsignal in Volt

Zeit in Minuten

EP 1 452 862 A1

Europäisches
Patentamt

**EUROPÄISCHER RECHERCHENBERICHT**

Nummer der Anmeldung

EP 04 10 0809

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| Y | FLEISCHER M ET AL: "Low-power gas sensors based on work-function measurement in low-cost hybrid flip-chip technology" SENSORS AND ACTUATORS B, ELSEVIER SEQUOIA S.A., LAUSANNE, CH, Bd. 80, Nr. 3, 1. Dezember 2001 (2001-12-01), Seiten 169-173, XP004311804 ISSN: 0925-4005 | 3,5 | G01N27/414 |
| A | * das ganze Dokument * | 1 | |
| | --- | | |
| Y | US 5 993 624 A (KANEKO SHINICHIRO  ET AL) 30. November 1999 (1999-11-30) * Spalte 3, Zeile 19-30 * * Spalte 9, Zeile 20 * * Spalte 12, Zeile 15-23 * | 3,5 | |
| | --- | | |
| A | DE 42 12 854 A (SIEMENS AG) 21. Oktober 1993 (1993-10-21) * Spalte 2, Zeile 33-68 * * Spalte 3, Zeile 14-17 * | 1,3,5-7 | |
| | --- | | RECHERCHIERTE SACHGEBIETE (Int.Cl.7) |
| A | EP 1 085 320 A (IMEC INTER UNI MICRO ELECTR) 21. März 2001 (2001-03-21) * Absätze [0002],[0003],[0031] * * Anspruch 10 * | 1,3 | G01N |
| | --- | | |
| A | EP 1 176 418 A (SIEMENS AG) 30. Januar 2002 (2002-01-30) * Absätze [0001],[0002],[0013],[0022],[0023],[0030] * | 1,3 | |
| | --- | | |
| | -/-- | | |

Der vorliegende Recherchenbericht wurde für alle Patentansprüche erstellt

| Recherchenort | Abschlußdatum der Recherche | Prüfer |
|---|---|---|
| MÜNCHEN | 1. Juni 2004 | Meyer, F |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder  Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument
    ...............................................
& : Mitglied der gleichen Patentfamilie, übereinstimmendes
    Dokument

EPO FORM 1503 03.82 (P04C03)

8

**Europäisches Patentamt**

# EUROPÄISCHER RECHERCHENBERICHT

Nummer der Anmeldung

EP 04 10 0809

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| A | LIAO B ET AL: "Study on CuO-BaTiO3 semiconductor CO2 sensor" SENSORS AND ACTUATORS B, ELSEVIER SEQUOIA S.A., LAUSANNE, CH, Bd. 80, Nr. 3, 1. Dezember 2001 (2001-12-01), Seiten 208-214, XP004311810 ISSN: 0925-4005 * Kapitel 2.1. * | 1-4,6,7 | |
| | --- | | |
| A | LEE M-S ET AL: "A new process for fabricating CO2-sensing layers based on BaTiO3 and additives" SENSORS AND ACTUATORS B, ELSEVIER SEQUOIA S.A., LAUSANNE, CH, Bd. 68, Nr. 1-3, 25. August 2000 (2000-08-25), Seiten 293-299, XP004216629 ISSN: 0925-4005 * Kapitel 2.1.-2.2. * | 1-7 | |
| | --- | | RECHERCHIERTE SACHGEBIETE (Int.Cl.7) |
| D,A | HAEUSLER A ET AL: "A novel thick film conductive type CO2 sensor" SENSORS AND ACTUATORS B, ELSEVIER SEQUOIA S.A., LAUSANNE, CH, Bd. 34, Nr. 1-3, 1. August 1996 (1996-08-01), Seiten 388-395, XP004078011 ISSN: 0925-4005 * das ganze Dokument * | 1-7 | |
| | --- | | |
| | -/-- | | |

Der vorliegende Recherchenbericht wurde für alle Patentansprüche erstellt

| Recherchenort | Abschlußdatum der Recherche | Prüfer |
|---|---|---|
| MÜNCHEN | 1. Juni 2004 | Meyer, F |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

**Europäisches Patentamt**

# EUROPÄISCHER RECHERCHENBERICHT

Nummer der Anmeldung

EP 04 10 0809

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| D,A | ISHIHARA T ET AL: "Improved sensitivity of CuO-BaTiO3 capacitive-type CO2 sensor by additives" SENSORS AND ACTUATORS B, ELSEVIER SEQUOIA S.A., LAUSANNE, CH, Bd. 28, Nr. 1, 1. Juli 1995 (1995-07-01), Seiten 49-54, XP004004388 ISSN: 0925-4005 * das ganze Dokument * ----- | 1-4,6,7 | |
| | | | RECHERCHIERTE SACHGEBIETE (Int.Cl.7) |

Der vorliegende Recherchenbericht wurde für alle Patentansprüche erstellt

| Recherchenort | Abschlußdatum der Recherche | Prüfer |
|---|---|---|
| MÜNCHEN | 1. Juni 2004 | Meyer, F |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
................................................................
& : Mitglied der gleichen Patentfamilie, übereinstimmendes Dokument

EPO FORM 1503 03.82 (P04C03)

**EP 1 452 862 A1**

## ANHANG ZUM EUROPÄISCHEN RECHERCHENBERICHT
## ÜBER DIE EUROPÄISCHE PATENTANMELDUNG NR.

EP 04 10 0809

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

01-06-2004

| Im Recherchenbericht angeführtes Patentdokument | | Datum der Veröffentlichung | Mitglied(er) der Patentfamilie | | Datum der Veröffentlichung |
|---|---|---|---|---|---|
| US 5993624 | A | 30-11-1999 | JP | 3376788 B2 | 10-02-2003 |
| | | | JP | 9159640 A | 20-06-1997 |
| | | | JP | 9264863 A | 07-10-1997 |
| | | | JP | 10073558 A | 17-03-1998 |
| DE 4212854 | A | 21-10-1993 | DE | 4212854 A1 | 21-10-1993 |
| EP 1085320 | A | 21-03-2001 | EP | 1085320 A1 | 21-03-2001 |
| | | | EP | 1085319 A1 | 21-03-2001 |
| | | | JP | 2001147215 A | 29-05-2001 |
| | | | US | 6521109 B1 | 18-02-2003 |
| EP 1176418 | A | 30-01-2002 | DE | 10036180 A1 | 14-02-2002 |
| | | | EP | 1176418 A2 | 30-01-2002 |

EPO FORM P0461

Für nähere Einzelheiten zu diesem Anhang : siehe Amtsblatt des Europäischen Patentamts, Nr.12/82

11